# EUROPEAN PATENT SPECIFICATION

(11) **EP 2 432 400 B1**
(45) Date of publication and mention of the grant of the patent: **28.08.2019**
(21) Application number: 09721524.8
(22) Date of filing: 17.03.2009
(51) Int. Cl.: A61B 17/04, A61B 17/115

(54) **SENSING ADJUNCT FOR SURGICAL STAPLERS**
DETEKTIONSZUBEHÖR FÜR CHIRURGISCHE KLAMMERGERÄTE
USTENSILE DE DÉTECTION POUR DES AGRAFEUSES CHIRURGICALES

(30) Priority: 13.03.2009 US 404276
(43) Date of publication of application: 28.03.2012
(73) Proprietor: Surgisense Corporation, Washington, DC 20007 (US); Zand, Jason, Washington, DC 20007 (US)
(72) Inventor: ZAND, Jason, Washington District Of Columbia 20007 (US); FISCHER, Gregory, Boston Massachusetts 02115 (US)
(74) Representative: Sutto, Luca
(86) International application number: PCT/IB2009/051135
(87) International publication number: WO 2009/115992

(56) References cited:
- WO-A2-2006/113394
- US-A- 5 518 163
- US-A1- 2004 111 018
- US-A1- 2005 131 390
- US-A1- 2009 054 908

## Description

### FIELD OF THE INVENTION

The present invention relates to surgical staplers, specifically to surgical staplers with sensors used to detect properties of biological tissue, and a system for exploiting the information gathered by the sensors.

### BACKGROUND ART

A living organism is made up of cells. Cells are the smallest structures capable of maintaining life and reproducing. Cells have differing structures to perform different tasks. A tissue is an organization of a great many similar cells with varying amounts and kinds of nonliving, intercellular substances between them. An organ is an organization of several different kinds of tissues so arranged that together they can perform a special function.

Surgery is defined as a branch of medicine concerned with diseases requiring operative procedures.

Although many surgical procedures are successful, there is always a chance of failure. Depending on the type of procedure these failures can result in pain, need for re-operation, extreme sickness, or death. At present there is no reliable method of predicting when a failure will occur. Most often the failure occurs after the surgical procedure has been completed. Failures of surgical procedures can take many forms. The most difficult failures to predict and avoid are those that involve biological tissue. This difficulty arises for three distinct reasons. Firstly, the properties that favor the continued function of biological tissue are very complex. Secondly, these properties are necessarily disrupted by surgical manipulation. Finally, the properties of biological tissues vary between patients.

During a surgical operation, a variety of surgical instruments are used to manipulate biological tissues. However, traditional surgical instruments do not have the ability to obtain information from biological tissues. Obtaining information from the biological tissues that surgical instruments manipulate can provide a valuable dataset that at present is not collected. For example, this dataset can quantitatively distinguish properties of tissues that will result in success or failure when adapted to specific patient characteristics.

Surgical instruments that incorporate sensors onto the instruments' working surfaces are described, e.g., in U.S. Patent Application No. 10/510,940 and in U.S. Patent 5,769,791. The instruments described in the prior art have the ability to sense tissue properties; however, their utility is limited by an inability to account for the multitude of differences that exist between patients. This limitation of the prior art is clearly illustrated by the fact that the instruments generate feedback after sensor signals are compared to a fixed dataset within the device. Thus, the prior art instruments have no means of adapting to patient-specific characteristics that are of utmost importance in avoiding surgical procedure failure. PCT Patent Application No. PCT/US2006/013985 describes a novel system and methodology for using the information gathered by surgical instruments having sensors in an adaptive, patient-specific manner.

Each surgical procedure has the potential for failure. A common procedure in gastrointestinal surgery is a bowel resection - removing the affected portion of the bowel and then mechanically joining the ends of the remaining segments to re-establish bowel continuity. The mechanical connection of the free ends of bowel forms what is termed a surgical anastomosis. A surgical anastomosis is formed by either traditional techniques using suture material, or by contemporary techniques utilizing surgical staplers. A surgical stapler mechanically joins the bowel by firing a pattern of staples from a cartridge or housing through the two free ends of bowel against an anvil that ultimately forms a securing crimp on the opposing side. There are many embodiments of surgical staplers. Some staplers form linear staple patterns, while others form circular patterns. Some staplers incorporate functionality for cutting tissue. Many staplers have the ability to vary the gap between the base of the staple and the formed crimp.

Anastomotic failure is one of the most feared complications of gastrointestinal surgery due to the resultant morbidity and mortality. Failure of an anastomosis, or intestinal junction, can cause a spectrum of morbidities to the patient including local abscess formation - requiring procedural drainage, tumor recurrence, debilitating pain, dysfunctional defecation, and overwhelming bacterial sepsis resulting in death. Despite improvements in surgical technique, there remains limited ability to assess the anastomotic segment and predict outcome, and as a result anastomotic failure occurs at unacceptably high levels given the severe consequences. For example, in the performance of a low anterior resection (LAR) for excision of rectal cancer, anastomotic failure has been reported to occur in up to 30% of cases. One large multicenter, observational study of 2729 patients reported a leak rate of 14.3%. These anastomotic failures cause a significant and avoidable economic burden on the healthcare system, as well as an incalculable amount of pain, suffering, and hardship for the patients in which the failure occurs. As a precaution many surgeons will avoid creating an anastomosis at the time of surgery and tunnel the free ends of the bowel through the abdominal wall to form a diverting stoma. The rationale of this maneuver is to prevent the leakage of fecal matter into the abdominal cavity from a potential anastomotic failure. Many times the surgeons will perform another procedure to reverse the stoma months after the initial procedure. In the same multicenter study 881 patients were given a temporary diverting stoma to mitigate the risk of an anastomotic leak, however, within this group only 128 patients developed a leak. Thus up to 85% of those patients underwent an additional surgical procedure to reverse the stoma that provided questionable benefit. The arbitrary creation of a temporary diverting stoma, and the eventual reversing procedure presents a significant and avoidable economic burden on the healthcare system, as well as exposes numerous patients to arguably unnecessary surgical risk. Presently, there is no reliable method or device available for predicting anastomotic failure, nor objective criteria by which to decide when a diverting stoma is indicated. Nor is there a device that can help to determine the optimal placement of an anastomosis.

There exists a need for a device, system and methodology for reducing anastomotic failures through the analysis of target tissues before, during, and after the creation of an anastomosis. There also exists a need to objectively determine, at the time of surgery, those patients that would benefit from a diverting stoma procedure. There also exists a need to deliver adjunct therapies to the anastomotic site to optimize outcome.

To accomplish its goal, the present invention couples to the desired stapling platform, which includes traditional off-the-shelf disposable surgical staplers, and uses an array of multimodality sensors to access the viability of the tissues at hand. If tissues are determined to be not suitable for an anastomosis, the present invention alerts the operative team to take corrective action, thus reducing the risk anastomotic failure. If after performing the anastomosis, the tissues are determined to be at high risk for failure, the present invention alerts the operative team to take corrective action.

One representative application of the present invention is in the treatment of colorectal cancer. Colorectal cancer (CRC) is the third most common cause of cancer for men and women in developed countries. Estimates predict that worldwide just under 1.2 million new cases of colorectal cancer were diagnosed in 2007. Rectal cancer accounts for approximately 27% of all colorectal cancers and presents the formidable challenge of ensuring a curative resection while maintaining acceptable function. The mainstay of treatment for rectal cancer is surgical resection - removing the affected portion of the bowel and performing an anastomosis on the ends of the remaining segments to re-establish bowel continuity. The end-to-end anastomosis (EEA) is most commonly performed using circular EEA staplers. As with any surgical procedure, resection of a rectal cancer can have complications. Amongst all of the possible complications the three most devastating to the patient in terms of morbidity and mortality are tumor recurrence, anastomotic leak and anastomotic stricture. Tumor recurrence can be reduced by: following oncologic principles of dissection, providing appropriate adjunctive chemotherapeutic, photodynamic, and radiation therapies, and preventing extra-luminal extravasation of residual intra-luminal neoplastic cells through anastomotic breakdown. Anastomotic failure has been anecdotally attributed to inadequate tissue perfusion and excessive tension at the anastomosis.

When determining the location of a rectal cancer the surgeon notes the distance of the tumor from the anal verge. The anal canal extends from 0-4cm past the anal verge, and the rectum 4-19cm. Surgically the rectum extends from the anal sphincters to the sacral promontory. The location of the cancer dictates the type of surgical procedure performed.

The primary goal of a curative resection is to remove all potential tissues harboring cancerous cells. To accomplish this goal, the surgical team aims to resect the tumor with a cancer free margin as well as the tumor's blood supply and draining lymphatic tissue. Tumors located in the upper rectum, greater than 12cm from the anal verge, are regularly amenable to an anterior resection (AR). Those in the mid rectum, between 6-12cm, are subject to a LAR with or without a total mesorectal excision (TME), and tumors in the lower rectum, 4-6cm, are usually treated with an ultra-low anterior resection (ULAR), incorporating a TME, and either a colorectal or coloanal anastomosis. A total mesorectal excision is a technique that attempts to resect the rectum and all investing soft tissues en-bloc. This technique has been touted in the literature as having superior results in terms of minimizing local tumor recurrence, however it is speculated that the procedure has an inverse effect on leak rates due to the excision of the supplying vasculature to the anastomotic site. Every attempt is made to retain fecal continence, however those tumors involving the anal sphincters 0-4cm are resected through a sphincter sacrificing abdominoperineal resection (APR).

As a secondary goal the surgical team strives to restore continuity of the bowel and ensuing fecal stream. To accomplish this goal an anastomosis is formed. Simply, an anastomosis is the surgical connection of two free ends of a tubular structure. When the continuity of the bowel cannot be restored, the fecal stream is diverted through a stoma, or opening, in the anterior abdominal wall through which the patient eliminates into an ostomy bag. There are two main reasons for stoma formation: resection of the anal sphincter complex, and diversion of the fecal stream. In a sphincter sacrificing procedure such as an APR, the patient is dependent on a permanent ostomy. With a sphincter sparing procedure such as a LAR, the fecal stream may diverted through a temporary ostomy in order to mitigate the risk of overwhelming sepsis resulting from fecal contents entering the abdominal cavity should there be a leak at the anastomosis. Most of the time a temporary stoma can be reversed within a few months after the initial operation through a separate procedure.

Anastomotic failure can cause a spectrum of morbidities to the patient including local abscess formation- requiring procedural drainage, tumor recurrence, debilitating pain, dysfunctional defecation, and overwhelming bacterial sepsis resulting in death. The scientific literature suggests that the cause of anastomotic failure is that inadequate tissue perfusion as a result of redefined vasculature, tissue interaction forces, edema, and tension result in a decrease of oxygen delivered to the anastomotic site. Without adequate oxygen delivery efficient aerobic cell respiration cannot occur within the native cells leading to tissue degradation, collagen matrix cannot mature into strong collagen fibrils, and white blood cells cannot effectively fight bacterial invasion.

### SUMMARY OF THE INVENTION

The present invention is defined in the claims. The present invention aims to reduce anastomotic failures through the analysis of target tissues before, during, and after the creation of an anastomosis. In one embodiment, the invention is an accessory for a circular stapler that allows measurement of tissue oxygenation, tissue perfusion, and tissue interaction force at twelve circumferential points around both sides of the staple joint. In one embodiment, the device incorporates three sensing modalities at each point: 1) tissue oxygenation using oximetry techniques, 2) tissue perfusion by measuring fluorescent response as fluorescein dye is injected, and 3) tissue interaction forces by utilizing MEMS pressure sensors. In addition, the staple gap height is concurrently monitored by an encoder mounted to the gap adjustment knob of the stapler. Monitoring the pressure and gap height during the creation of anastomosis provides consistency among procedures. An independent reference module with the same or similar array of sensing elements can be used concurrently to establish a baseline measurement at a tissue location independent from the surgical site.

If tissues are determined to be unsuitable for an anastomosis, the present invention alerts the operative team to take corrective action to limit the chance of anastomotic failure. Corrective actions may include any combination of the following: optimizing the location of the planned anastomosis, creating a temporary or permanent stoma, changing the operative procedure, or delivering adjunct therapies to enhance outcome.

Accordingly, several objects and advantages of the present invention are:
To provide an adjunct to a surgical stapler, where the adjunct is configured with at least one sensor that can operate independently of said stapler. The adjunct can take the form of an optionally coupled accessory to a surgical stapler, or a stand-alone substitutive component acting to serve as a replacement for a component of the surgical stapler such as an anvil.
To provide the described functionality without disrupting the surgical workflow.
To provide a sensing adjunct to a surgical stapler that is communicatively coupled to a controller or base station by wireless methods.
To reduce patient morbidity, and mortality secondary to complications, or additional procedures related to intestinal, or other tissue anastomosis.
To reduce surgical anastomotic failures, and improve outcome though the evaluation and analysis of tissues at the anastomotic site prior to, during, and after the creation of the anastomosis.
To provide the operative team an assessment of the viability of a surgical anastomosis.
To provide signal to the operative team when the creation of a diverting stoma, or change in surgical procedure is indicated.
To extend the capabilities of standard non-sensing surgical staplers to enable the sensing and analysis of subject tissues.
To enable the consistency of surgical anastomoses.
To deliver adjunct therapies to the anastomotic site.
To quantitatively monitor the staple gap or height.
To automatically adjust, or provide a signal to the operative team to adjust the staple gap or height responsive to a monitored parameter.
To assist in the firing of a surgical stapler.
To extend the capabilities of standard non-sensing surgical staplers to enable the monitoring, and / or adjustment of a staple gap or height.
To extend the capabilities of standard non-sensing surgical staplers to enable the delivery of adjunct therapies to subject tissues.
To extend the capabilities of standard non-sensing surgical staplers to enable the deployment of a tissue monitor to the anastomotic site such as described in PCT Patent Application No. PCT/US2007/071718.
To incorporate a reference module for the baseline comparison of subject tissues.
To enable tissue interrogation to generate a signal indicative of a tissue property, independently, or in conjunction with other components of the present invention.
To provide for the described functionalities in a durable, limited lifetime, or disposable manner.
To interact with a system which generates real time, patient specific procedural guidance for predicting success of a surgical anastomosis, and avoiding or detecting failure of the surgical anastomosis. Another advantage of the present invention is a system which records data across the entire patient encounter including pre-operative, intra-operative and post-operative periods, as well as immediate, acute, short term, and long term outcomes both locally in hospital-based units as well as remotely in a data repository as described in PCT Patent Application No. PCT/US2006/013985.

Additional advantages of the present invention will become readily apparent to those skilled in this art from the following detailed description, wherein only selected embodiments of the present invention are shown and described. As will be realized, the present invention is capable of other and different embodiments, and its several details are capable of modifications in various obvious respects, all without departing from the invention. Accordingly, the drawings and description are to be regarded as illustrative in nature, and not as restrictive.

### BRIEF DESCRIPTION OF THE DRAWINGS

Reference is made to the attached drawings, wherein elements having the same reference numeral designations represent like elements throughout, and wherein:
FIG. 1 shows the system overview
FIG. 2 shows a dual-sided sensing stapler configuration
FIG. 3 shows an anvil-only configuration of a sensing stapler
FIG. 4 shows one embodiment of a sensing anvil accessory with light guides
FIG. 5 shows one embodiment of a sensing anvil with light guides
FIG. 6 shows one embodiment of a sensing anvil with integrated pressure sensing
FIG. 7 shows one embodiment of a sensing anvil with pressure sensing at discrete points
FIG. 8 shows one embodiment of a sensing anvil accessory with sensors at the surface
FIG. 9 shows one embodiment of a sensing housing accessory with light guides
FIG. 10 shows one embodiment of a sensing housing accessory with sensors at the surface
FIG. 11 shows one embodiment of a postoperative monitor
FIG. 12 shows another embodiment of a postoperative monitor
FIG. 13 shows one embodiment of a reference sensor
FIG. 14 shows a flowchart for one embodiment of the system use in a procedure

### DESCRIPTION OF THE INVENTION

The present invention relates to an adjunct or accessory to a surgical stapler, where the adjunct is configured with at least one sensor that can operate independently of said stapler. The adjunct can take the form of an optionally coupled accessory to a surgical stapler, or a stand-alone substitutive component acting to serve as a replacement for a component of the surgical stapler such as an anvil. The sensors may sense mechanical or biological properties. The sensing modalities may include mechanical, optical, chemical, electrical, or other means for generating a signal indicative of a property of a subject tissue. The sensors are incorporated into or coupled to the working surface (i.e. tissue contacting surface) of an adjunct to a surgical stapler. The sensors may be incorporated into a separate accessory for the stapler affixed to either or both the anvil and housing sides of the stapler. The sensors act independently, are communicatively coupled to each other and/or a base station wirelessly. The sensors are powered by an onboard battery, capacitor, or other power storage element. Alternatively, the sensors are powered by an external source, including, but not limited to, inductive coupling, radio frequency (RF) energy, or mechanical motion.

In one embodiment of the present invention, banks of sensors are placed around the periphery of the staple line on a circular stapler. The sensors are located on one or both sides of the stapler (anvil and housing). The banks of sensors are incorporated into an accessory (i.e. a replacement part such as the anvil, or a cap/shell that optionally couples to an existing component of the stapler). The bank of sensors contains mechanical and optical sensing modalities. Mechanical sensing includes, but is not limited to, pressure sensors that monitor tissue interaction forces including compression pressure and tissue tension. Optical sensors include but are not limited to light emitters including light emitting diodes (LEDs) and laser diodes, and light receivers including photodiodes, CCD arrays, CMOS sensors, and spectrometers. The optical sensors are configured to measure at least one of tissue oxygenation, oxygen delivery, oxygen utilization, tissue characterization, and tissue general health using oximetry, or spectroscopic techniques, and at least one of tissue perfusion, tissue flow dynamics, tissue oxygen content, tissue chemical composition, tissue immunologic activity, tissue pathogen concentration, or tissue water content using fluorescence based techniques. The fluorescence based techniques include but are not limited to the following: monitoring and analyzing the intensity and time course of a fluorescent response responsive to the injection or activation of a fluorescent medium, determining oxygen quantities by measuring oxygen quenching of fluorescent radiation using a sensitive material such as Ruthenium by both intensity and time resolved methods, and determining the target tissue property by quantitative fluorescent methods including the use of quantum dots, or other fluorescent based biomarkers.

An additional element of the present invention is the incorporation of an independent reference sensor. The reference sensor contains one or more sensor banks of the same or similar type to those sensors on the working surface of the stapler. One or more reference sensors may be placed on one or more portions of the tissue to serve as a baseline measurement. In one instance, the reference sensor is placed on a healthy portion of bowel and remains in place while an anastomosis is performed on a different site. The reference may also be used as an independent sensor for preoperative or postoperative monitoring, or selection of a surgical site.

A further element of the present invention is the incorporation of a sensor that reports the staple gap or relative change thereof. In one embodiment, a sensing module is placed over the gap adjustment knob on a standard surgical stapler. The sensor incorporates an optical encoder module that interacts with a reflective encoder surface mounted to the base of the surgical stapler.

A further element of the present invention is the incorporation of a motor actuated staple gap adjustment module that will vary the staple gap responsive to at least one of user, or sensor input. The sensor input includes, but is not limited, to tissue oxygenation, compression pressure, or staple gap height. In one embodiment the motor actuated gap adjustment module is placed over the gap adjustment knob / end of a standard surgical stapler.

A further element of the present invention is the incorporation of a motor actuated stapler firing module which will fire the stapler responsive to at least one of user input, or sensor based input.

In another embodiment of the present invention, implantable sensors are placed at the surgical site to perform post-operative monitoring. The sensors may be tethered by electrical or fiber optic means, or may be wireless. Wireless sensors may be powered by an onboard source or external source.

In a further embodiment, the anvil and/or housing can act as a delivery mechanism for doses of adjunct therapies not limited to photodynamic, pharmaceutical, bio-adhesive, brachy-, and nano- therapies. The light sources used for optical sensing may also serve as an agent for activating light-sensitive treatment such as in photodynamic therapy (PDT). Alternatively the adjunct therapies can be delivered from the device to the target site through microtubules, needles, micro-fluidic methods, diffusion, and conduction.

In a further embodiment the reference module can act as a stand-alone tissue interrogator to determine tissue viability, or suitability for a particular procedure. The tissue interrogator can take the form of an instrument used in open or minimally invasive surgery. One representative application of the tissue interrogator embodiment is in the performance of a bowel resection secondary to a bowel obstruction. Often a bowel obstruction is caused by an adhesion which causes the bowel to twist upon itself or a hernia which incarcerates or strangulates the bowel. The blood supply to the bowel can be compromised leading to ischemia, or infarction. Intraoperatively, the surgeon qualitatively determines if the bowel is viable after it has been untwisted or freed. If the bowel does not appear viable the bowel is resected. Often qualitative methods are not accurate in determining bowel viability. An advantage of the present invention is the ability to quantitatively assess bowel viability and ensure the anastomotic joint is performed on tissue that has a minimum chance of failing due to inadequate perfusion.

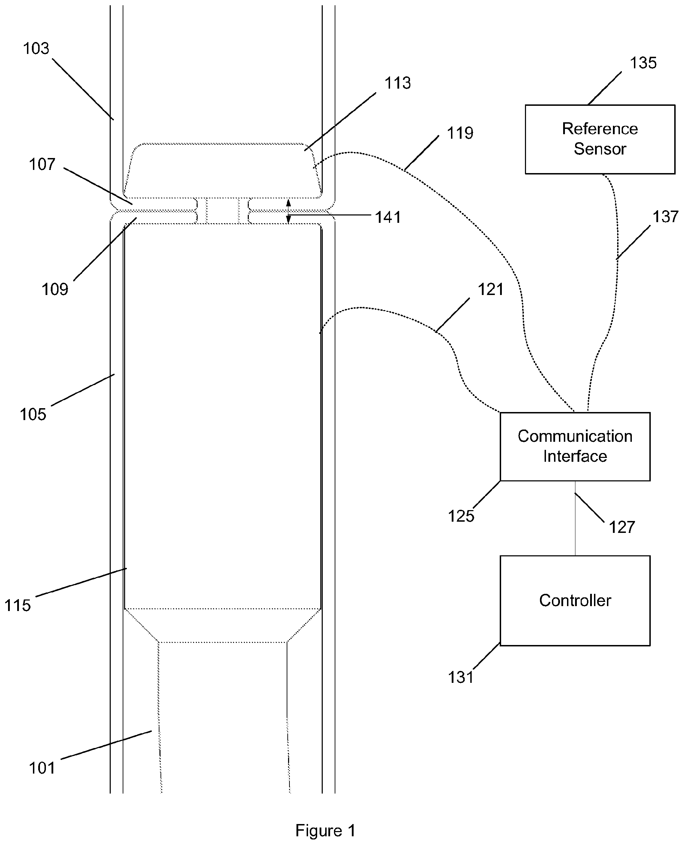

The present invention will now be described in detail with reference to Figs. 1-14.

FIG. 1 shows a representative surgical stapler augmented with sensing capabilities according to an embodiment of the present invention. This embodiment specifically depicts the augmented surgical stapler 101 for measuring properties of bowel tissue 103 and 105. Tissues 103 and 105 may represent other tissues being joined at an anastomotic site. Measurements are made at the site of the surgical anastomosis on tissue 107 and 109. The anvil sensing adjunct 113 takes measurements from the distal side of tissue 107. The housing sensing adjunct 115 takes measurements of proximal tissue 109. The sensing adjuncts 113 and 115 are coupled via wireless connections 119 and 121 respectively to a communication interface 125; alternatively the sensing adjuncts can function independently. A further reference sensor device 135 is placed outside the surgical area to capture baseline measurements. Reference sensor device 135 communicates via wireless link 137 to communication interface 125. A further gap sensing adjunct monitors the spacing between the anvil 113 and housing 115 tissue contacting surfaces to determine the staple gap height 141 and the compression of tissues 107 and 109. The gap sensing adjunct communicates wirelessly with communication interface 125. Alternatively, the sensing devices may communicate with each other as a sensor network. Further one sensing device may act as a hub that relays information to the communication interface 125. Communication interface 125 is communicatively coupled 127 to the controller 131. Controller 131 may act as a stand-alone device, or it may be connected to a database to evaluate current sensor readings responsive to past experiences. Data and outcomes may also be recorded internally or to an external database.

Depending on the sensing modality, the sensor data is translated into information that relates to tissue properties. In one exemplary optical sensing embodiment, oximetry-type techniques are used to convert the relative absorption of different wavelengths of light into an oxygen saturation percentage of hemoglobin in the blood. In another optical sensing modality, fluorescence response due to a fluorescent medium that has been introduced into the body is measured, and characteristics of the response including the intensity, rise time, and steady state value are indicative of the blood flow in the tissue in question. Further, compression pressure, and tension on tissues 107 and 109 can be measured at discrete or distributed sites on sensing adjuncts 113 and 115.

In a further embodiment of the system an adjunct or surgical stapler may be configured to deliver or regulate photodynamic or other therapy.

In a further embodiment of the system an adjunct assists in the determination of and automated or interactive adjustment of staple gap.

In a further embodiment of the system an adjunct assists in the firing of the surgical stapler.

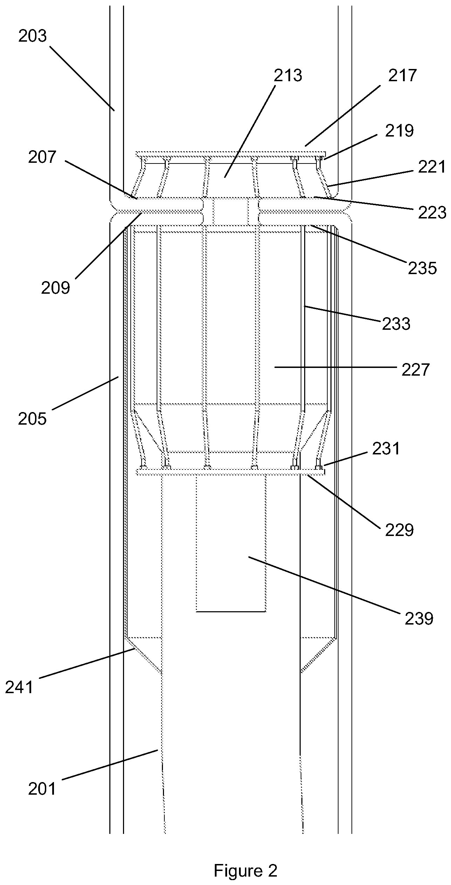

FIG. 2 depicts a specific configuration of a stapler augmented with sensing capabilities where sensing adjuncts are on both sides of the surgical staple joint. Surgical stapler 201 joins bowel or other segments 203 and 205. The anastomosis is formed by joining tissue flaps 207 and 209. The stapler's anvil 213 lies on the distal end of the anastomosis. Control board and power source 217 lie on anvil 213. Sensor banks 219 are on the face of control board 217. Optical emitters and receivers 219 are coupled optically through light guides or optical fibers 221 to the working surface of the instrument 223 to monitor tissue 207. The described components of the anvil sensing adjunct are enclosed in shell that is optionally coupled to anvil 213.

The stapler's housing 227 lies on the proximal end of the anastomosis. Control board and power source 229 lie on housing 227. Sensor banks 231 are on the face of control board 229. Optical emitters and receivers 231 are coupled optically through light guides or optical fibers 233 to the working surface of the instrument 235 to monitor tissue 209. Additional control board or power source 239 lies on stapler shaft 201 to provide for additional functionality. The described components of housing sensing adjunct are enclosed in shell 241.

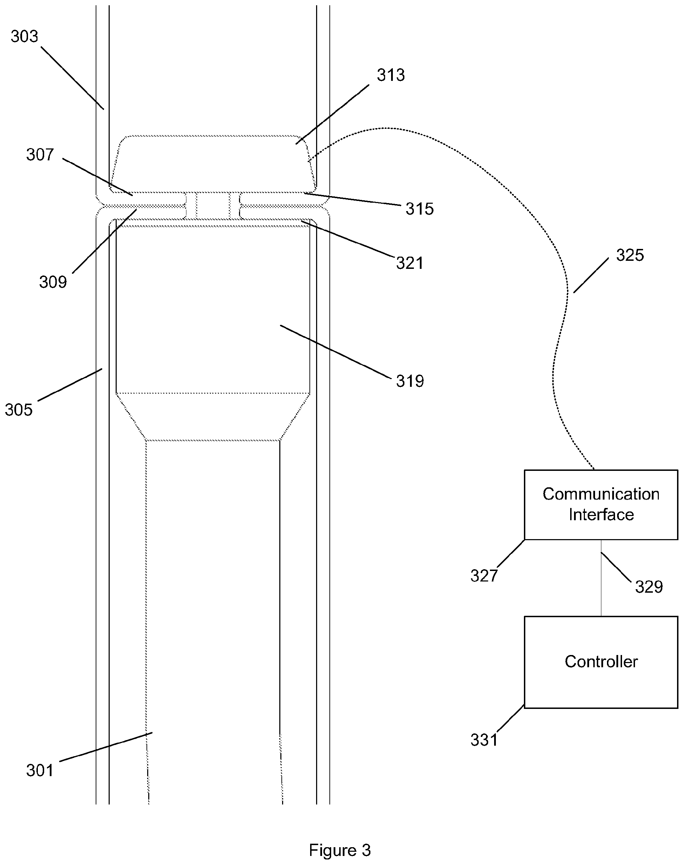

FIG. 3 depicts a configuration of a surgical stapler augmented with sensing capabilities located only on the anvil side. The sensor device may take the form of an optionally coupled adjunct to, or serve as a complete replacement of a standard non-sensing anvil of a surgical stapler. Circular surgical stapler 301 joins bowel or other tissue segments 303 and 305. The anastomosis is formed by joining tissue flaps 307 and 309. The stapler's anvil 313 lies on the distal end of the anastomosis. The anvil sensor device incorporates sensing elements to monitor tissues 307 and 309 between the tissue contacting surfaces 315 and 321. Sensing depth may be modulated to distinguish tissue properties of either side 307, or side 309, or both 307 and 309. Stapler housing 319 and the remainder of stapler 301 need no modification from a standard EEA surgical stapling product. Anvil sensing adjunct 313 is communicatively coupled via 325 to communication interface 327 that is in turn communicatively coupled via 329 to controller 331.

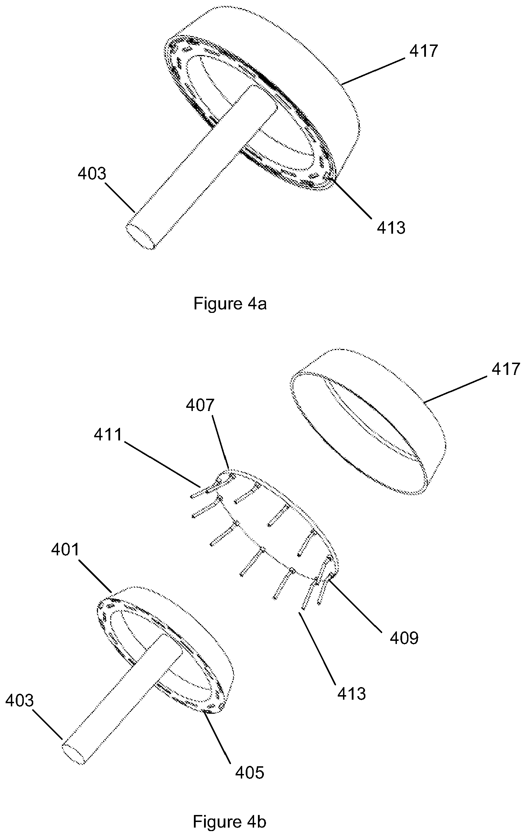

FIG. 4 depicts one embodiment of an anvil sensing adjunct that may present as either an optionally coupled adjunct to, or serve as a complete replacement of a standard non-sensing anvil of a surgical stapler. Anvil 401 couples to the stapler via shaft 403. Staple forms 405 are embedded within the working surface of anvil 401. Control board 407 incorporates sensors 409. Optical sensors and emitters 409 are optically coupled to the working surface via light guides or optical fibers 411. Tips 413 may be shaped to provide appropriate light distribution or specially coated to provide extended sensing capabilities such as determining oxygen quantities by measuring oxygen quenching of fluorescent radiation. The device is sealed inside cap 417.

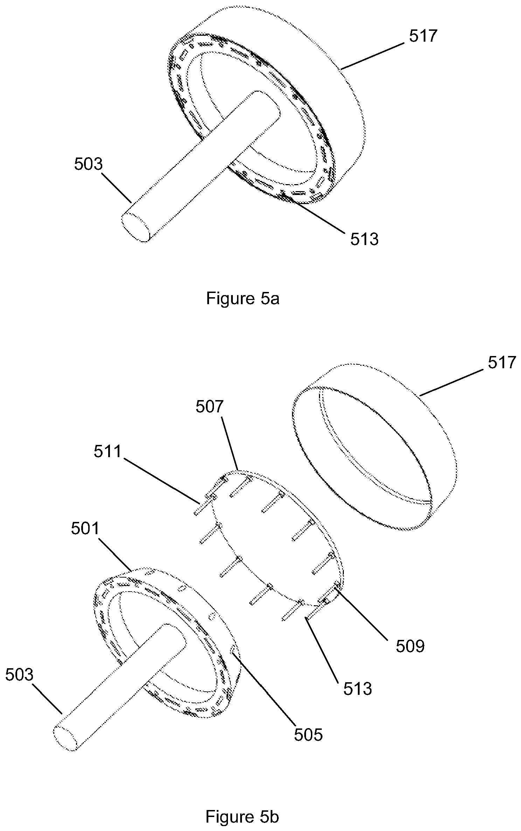

FIG. 5 depicts a further embodiment of an anvil sensing adjunct. Anvil 501 couples to the stapler via shaft 503. Guide-ways 505 provide a path from control board 507 to the working surface of anvil 501 between staple forms 506. Control board 507 incorporates sensors 509. Optical sensors and emitters 509 are optically coupled to the working surface via light guides or optical fibers 513. Tips 513 may be shaped to provide appropriate light distribution or specially coated to provide extended sensing capabilities. Pressure sensors 509 are mechanically coupled by shafts 511 to the working surface to resolve compression forces. The device is sealed inside cap 517.

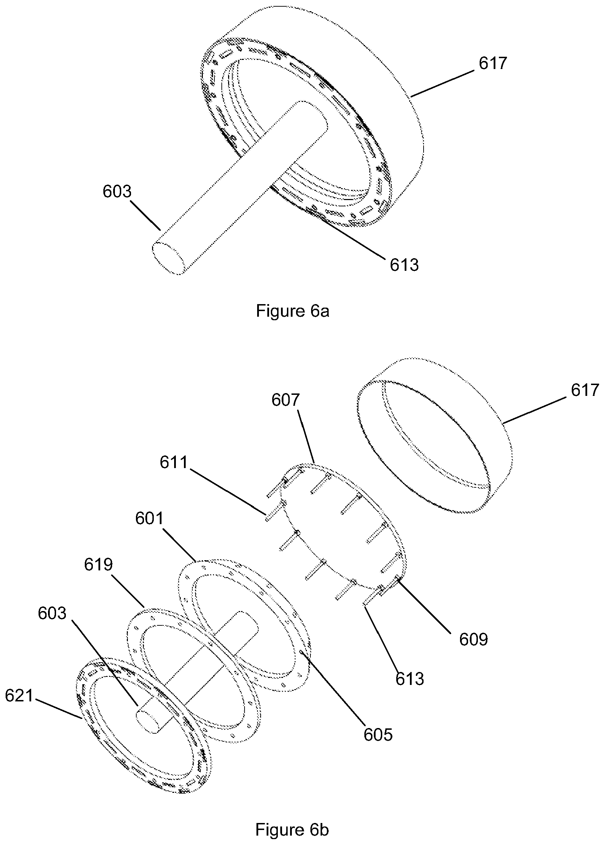

FIG. 6 depicts a further embodiment of an anvil sensing adjunct. Anvil base 601 couples to the stapler via shaft 603. Guide-ways 605 provide a path from control board 607 to the working surface of anvil staple form plate 621. Control board 607 incorporates sensors 609. Optical sensors and emitters 609 are optically coupled to the working surface via light guides or optical fibers 613. Tips 613 may be shaped to provide appropriate light distribution or specially coated to provide extended sensing capabilities. Pressure sensor element 619 measures compression force between anvil base 601 and staple form plate 621 and is communicatively coupled to control board 607. The device is sealed inside cap 617.

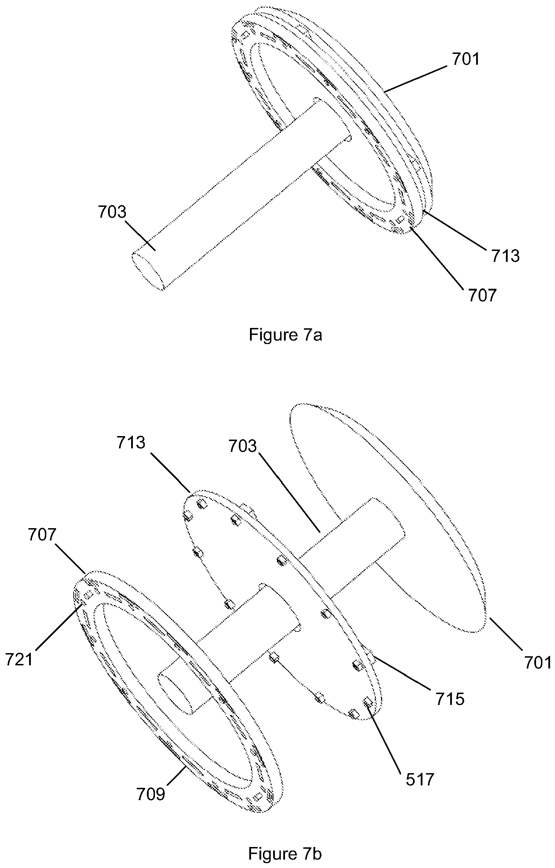

FIG. 7 depicts a further embodiment of an anvil sensing adjunct. Anvil base 701 couples to the stapler via shaft 703. Anvil staple form plate 707 is compressed against the tissue at the working surface 709 by anvil base 701 through control board 713. Attached to control board 713 are pressure sensors 715 on the distal surface and optical sensors 717 on the proximal surface. Optical sensors and emitters 717 lie on staple form plate 707 and are aligned with openings 721. Optical emitters and sensors transmit light and receive light respectively through these openings. The openings can be filled with an optical potting compound or light guides. Control board 713 provides a single electronics board that contains elements for both optical tissue property sensing and compression force sensing. A power source may be incorporated in control board 713 or attached on the distal end of anvil base 701. The power source may be a battery or other source of power. The power source may be single use or rechargeable. Charging may be through a direct electrical connection or an inductive coupling. The components of the anvil sensing adjunct are sealed inside a cap, or alternatively sealed by over-molding or other encapsulation.

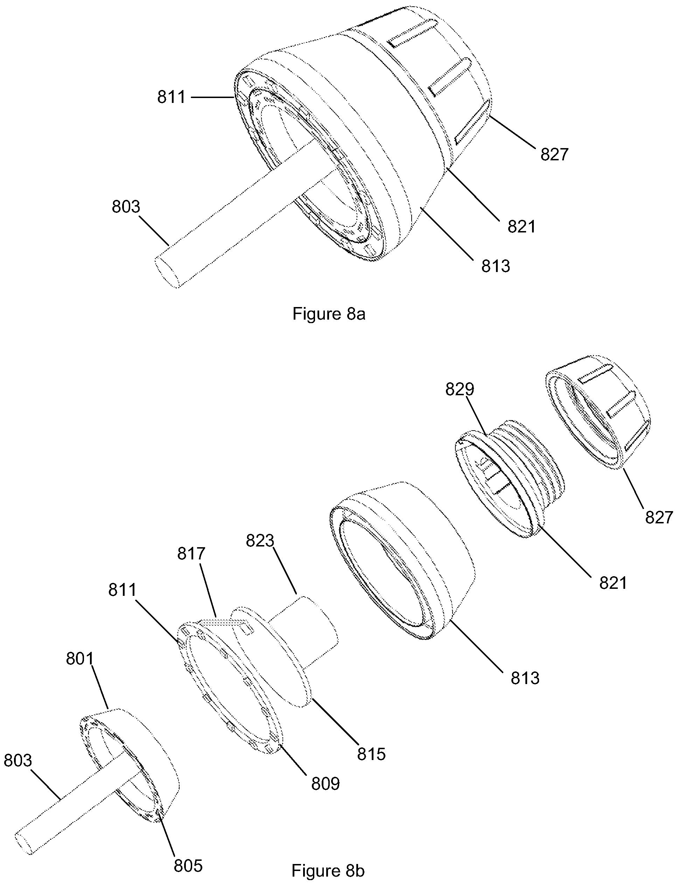

FIG. 8 depicts a further embodiment of an anvil sensing adjunct. The sensing adjunct is an accessory to a standard anvil 801. Anvil 801 couples to the stapler via shaft 803. The working surface (tissue contacting surface) with staple forms 805 contacts tissue at the distal surface of the anastomosis. Sensor ring 809 contains sensor elements 811. Sensor ring 811 fits within lower shell 813 and maintains the sensors 811 flush with working surface 805. In one embodiment, sensor ring contains twelve sets of sensors each including LEDs, photodiodes, and pressure sensors. Surface of sensor ring 811 is sealed with a thin layer of optical encapsulate. In another embodiment the photodiodes or light guides optically coupled to the photodiodes may extend directly to the working surface 805. In this embodiment the surface of the photodiode or light guide may be shaped to provide appropriate light distribution or specially coated to provide extended sensing capabilities such as determining oxygen quantities by measuring oxygen quenching of fluorescent radiation. Flexible cable 817 couples sensor ring 809 to control board 815 mounted within lower housing 813. Control board 815 contains a processor, wireless communications interface, memory, power regulator, led driver, analog sensor input and indicators. Upper shell 821 covers and seals control board 815 and provides a screw thread for cap 827. Battery 823 powers the sensor and is replaceable by removing cap 827. Cap 827 seals the sensor with o-ring 829.

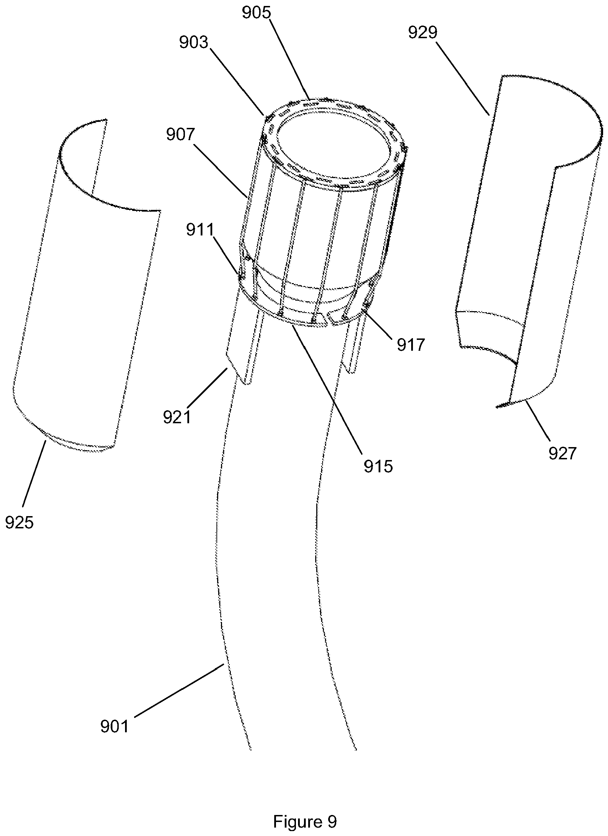

FIG. 9 depicts an embodiment of a housing sensing adjunct. Stapler 901 contacts the tissue at the proximal side of the anastomotic junction at surface 905. In one embodiment, the adjunct is split into halves. The sensor boards 915 and 917 contain sensor elements 911. These sensor elements may contain optical sensors incorporating LEDs and photodiodes. Control boards 921 extend from 915 and 917 to provide additional space for electronics, communications, and power sources. Optical sensor elements (including optical emitters and receivers) 911 are coupled to the working surface 903 by light guides or optical fibers 907. The tips 903 may be shaped to provide appropriate light distribution or specially coated to provide extended sensing capabilities such as determining oxygen quantities by measuring oxygen quenching of fluorescent radiation. Shell halves 925 and 927 enclose the described sensor components. The two halves are sealed independently and joined at hinge joint 929. The two halves close around stapler 901.

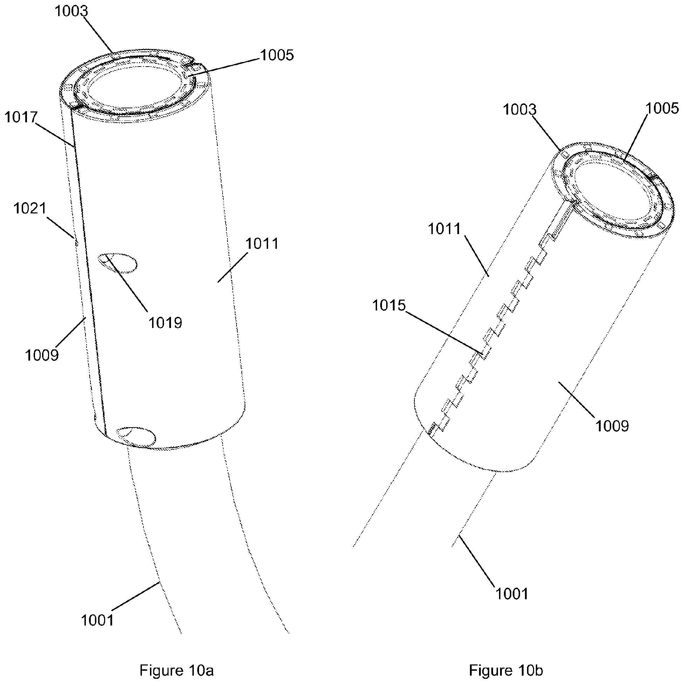

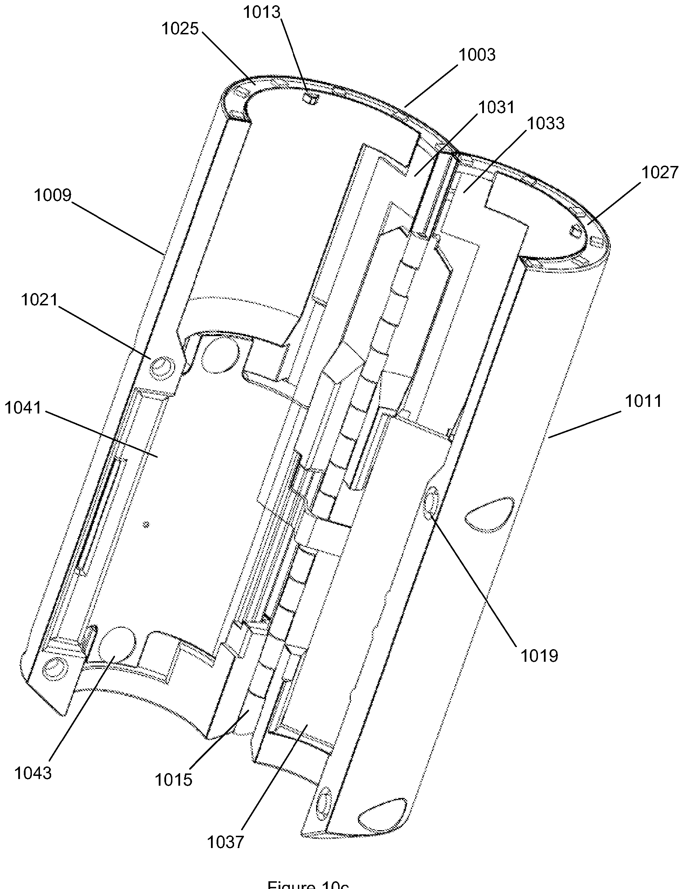

FIG. 10 depicts a further embodiment of a housing sensing adjunct. Stapler 1001 contacts the tissue at the proximal side of the anastomotic junction at surface 1003. Sensor banks 1005 lie flush with the working surface of the stapler 1003. The sensor is split into two halves 1009 and 1011. The two shells 1009 and 1011 are connected at hinge joint 1015 and close around stapler 1001. Tabs 1013 ensure alignment of the sensor to the stapler. The housing sensing adjunct is optionally coupled to the stapler 1001 with screws 1019 and nuts 1021 or a clip or other fastening device. Sensor boards 1025 and 1027 lie inside the top cavity of shells 1009 and 1011. Sensor elements 1005 lie on top of sensor boards 1025 and 1027. In one embodiment, sensor boards 1025 and 1027 contain six sets of sensor banks, each incorporating LEDs, photodiodes, and pressure sensors. In one embodiment the surface of sensor boards 1025 and 1027 is sealed with a thin layer of optical encapsulate. In another embodiment the photodiodes or light guides optically coupled to the photodiodes may extend directly to the working surface 1003. In this embodiment the surface of the photodiode or light guide may be shaped to provide appropriate light distribution or specially coated to provide extended sensing capabilities such as determining oxygen quantities by measuring oxygen quenching of fluorescent radiation. Flexible cables 1031 and 1033 connect sensor boards 1025 and 1027 to control board 1037. Control board 1037 may be a flexible circuit board fit within shells 1009 and 1011. Battery 1041 fits within shell 1009 and locks in place with screws or latches 1043. Battery 1041 seals against the flex cables preventing and leaks when locked in place. All other components are fully sealed.

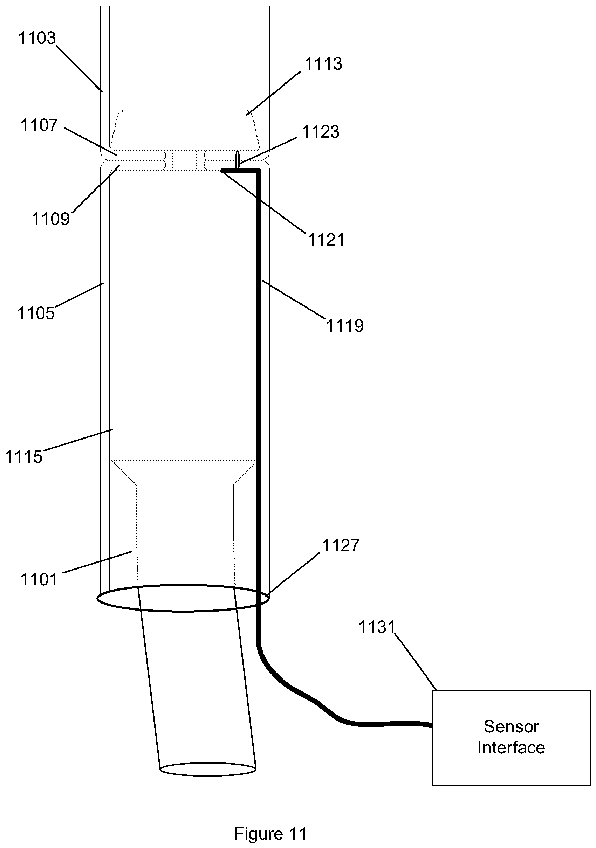

FIG. 11 depicts a sensor for intra-operative and post-operative monitoring of tissue, specifically tissue at an anastomotic junction. In one embodiment, stapler 1101 joins bowel segments 1103 and 1105. Tissue flaps 1107 and 1109 are joined at the staple joint between stapler anvil 1113 and stapler housing 1115. Stapler anvil 1113 and stapler housing 1115 may be traditional components, or part of a sensing surgical stapler. Connection 1119 couples sensor tip 1121 to sensor interface 1131. Connection 1119 may be an electrical connection to compact sensor 1121, or it may be a fiber optic connection to sensor tip 1121. In one embodiment, sensor 1121 is mounted on a bioabsorbable patch that is stapled into place during the anastomosis by staples 1123. In another embodiment, sensor 1121 is fastened by a bioabsorbable clip 1123. In the case of an anastomotic junction of the large bowel, connection 1119 passes out the anus 1127 to sensor interface 1131. Connection 1119 may be bioabsorbable such that it breaks away after a period of time. Alternatively, connection 1119 may be able to be pulled to cause the sensing tip to detach from the sensing site.

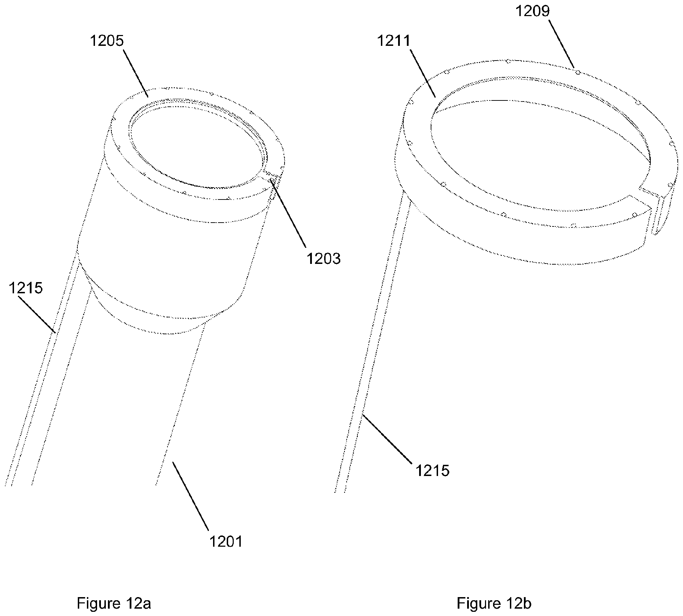

FIG. 12 depicts an alternate embodiment for and intra-operative and post-operative tissue monitor. Stapler housing 1201 performs staple joint at surface 1203. Sensor clip 1205 sits on stapler surface 1203. Sensing elements 1209 monitor tissue properties circumferentially around the staple joint. Flap 1211 lies over stapling surface 1203 and is fastened in place as the anastomosis is formed. The material of 1211 may be bioabsorbable. Optical fibers or other connection 1215 couple the sensing elements 1209 to a sensor interface. The gap in 1205 allows radial expansion of the anastomotic joint to allow for expansion of the bowel lumen so that the anvil and fecal matter may pass. Similarly, one or more sensors can be placed circumferentially on the tissue contacting surface of an endoluminal compression anastomosis ring.

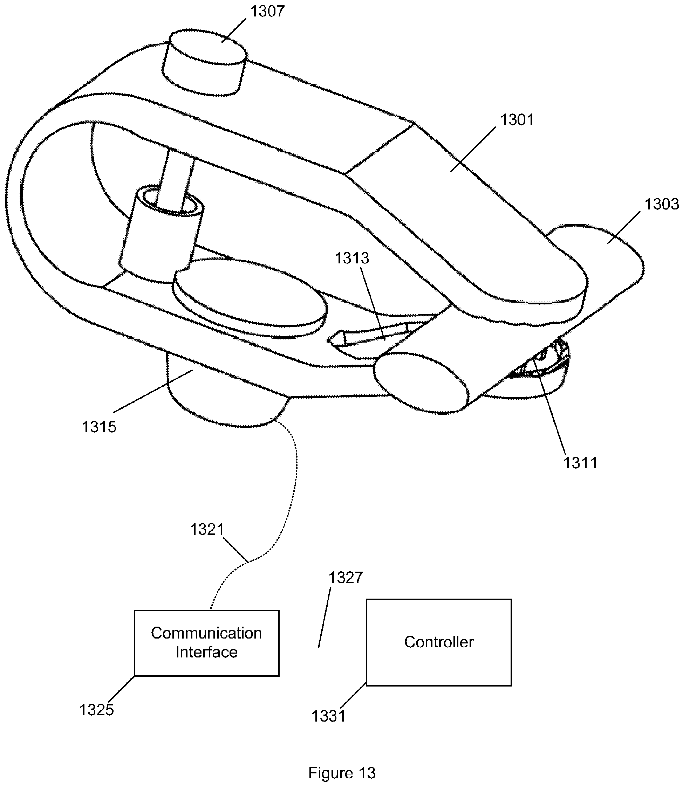

FIG. 13 depicts one embodiment of a reference sensor device. The reference sensor device 1301 clamps on tissue 1303. In one embodiment, tissue 1303 is healthy bowel tissue used as a baseline measurement from which to compare sensor readings at the surgical site. In another embodiment, reference sensor is used to interrogate tissue and determine tissue viability for planning or monitoring of a procedure. Knob 1307 adjusts the clamping pressure on tissue 1303. Sensors 1311 interrogate the tissue. These sensors may be the same as those on the corresponding sensing surgical instrument or of another type. In one embodiment, two sets of sensors 1311 include photodiodes, LEDs, and pressure sensors for the purpose of measuring tissue oxygen saturation, blood perfusion, and compression pressure on the tissue. Flexible circuit board 1313 supports the sensors 1311 and couples them to the control board within cavity 1315. Cap 1317 seals in the control board and power source. In one embodiment, reference sensor 1301 is communicatively coupled via a wireless link 1321 to communication interface 1325, which is in turn coupled via 1327 to controller 1331. Controller 1331 may receive signals from one or more reference sensors and other sensor devices to make an assessment of the surgical procedure. Alternatively, reference 1301 may directly communicate wirelessly with other sensor devices in a stand-alone mode. In the stand-alone mode with no external display, indicators on the reference provide feedback to the operative team responsive to the tissue analysis.

In a further embodiment a sensing device may be used independently to interrogate subject tissue to determine a physiologic or physical property such as a bowel ischemia.

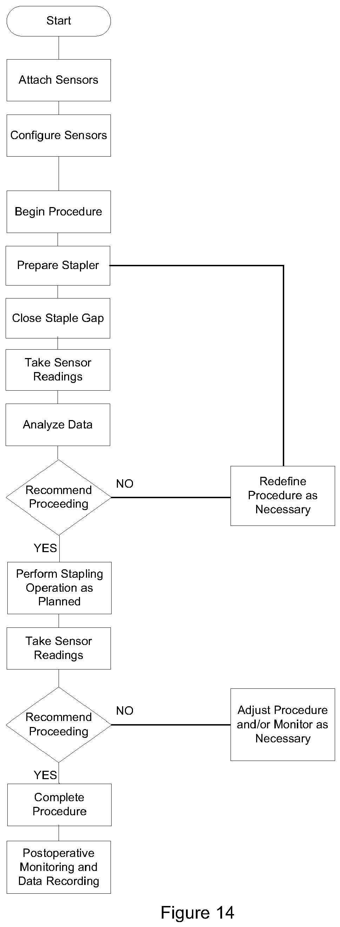

FIG. 14 is a flowchart for one inventive embodiment of the system comprising sensing adjuncts coupled to surgical instruments. Sensing adjuncts are coupled to the surgical instruments and the reference sensor is placed. The sensors are configured appropriately. The stapler is placed at the surgical site and gap is closed. Sensor readings are obtained and analyzed, optionally responsive to a reference sensor or database. The system indicates the likelihood of success of the procedure with the current conditions. The procedure can be adjusted or performed as planned. After performing the procedure, such as a circular anastomosis of bowel tissue, further sensor readings are taken. Sensor readings are analyzed and the system indicates the likelihood of a successful outcome based on the current conditions. The operative team may make adjustments to the operative plan, or complete the procedure. Optionally sensors may be placed at the surgical site for continuous post-operative monitoring.

The optical sensors described in the embodiments may take one several forms. In one form, light emitting diodes or other light sources illuminate tissue and light receivers receive the signal. The light receivers can be photodiodes, photodiode arrays, avalanche photodiodes, photomultiplier tubes, linear and two dimensional CCDs, CMOS sensors, spectrometers, or other sensor types. This may be used for oximetry or spectroscopy type measurements. Wavelengths centered near 660nm, 800nm, and 950nm are used in one embodiment for oximetry measurements; other combinations of two or more wavelengths may also be used. In another embodiment, wavelengths near 680nm 720nm, 760nm, and 800nm are used to make measurements based on first or second derivative spectroscopy; other discrete wavelengths or a multispectral light source may also be used to generate the differential or second differential spectroscopy signal. Additionally, light sources may be used to illuminate tissue infused with a fluorescent dye to monitor perfusion. This monitoring may be done by either an intensity based measurement or a time-resolved measurement. In a further embodiment, the tips of sensors may be coated with a material such as Ruthenium to determine oxygen quantities by measuring oxygen quenching of fluorescent radiation. The coating may be illuminated to cause it to fluoresce and measurements may be made on intensity-based or time-resolved measurements.

The sensors described in the embodiments above can be used to perform said oximetry-type and/or fluorescence-type sensing. These techniques can be combined with other sensing modalities including optical sensors, electrical sensors, chemical sensors, mechanical sensors, electro-mechanical sensors, MEMS sensors, nano sensors, biochemical sensors, acoustic sensors, immunologic sensors, fluidic sensors, micro-dialysis based sensors or other types of sensors. The sensors may sense electrical properties, chemical properties, general health, tissue oxygenation, blood oxygenation, pulse rate, pulse presence, pulse rhythm, tissue perfusion, staple gap, compression force, tissue interaction force, staple tension, grasping forces, fluorescence, tissue electrical impedance, tissue electrical activity, pH, concentration of cellular respiration metabolites (e.g. lactic acid), electromyography, temperature, fluid flow rate, fluid flow volume, tissue pressure, blood pressure, biomarkers, radiotracers, immunologic characteristics, biochemical characteristics, nerve activity, an evoked potential, oxygen delivery, oxygen utilization, tissue characterization, tissue general health, tissue flow dynamics, tissue chemical composition, tissue immunologic activity, tissue pathogen concentration, tissue water content, blood hemoglobin content, tissue chromophore content (e.g. hemoglobin), tissue neoplastic cell content and tissue dysplastic cell content, and/or other parameters. The sensing modalities may be implemented using techniques known to those skilled in the art.

The embodiments described above demonstrate how the sensing adjuncts integrate with a circular stapler. These embodiments are for meant as illustrative purposes. The sensing adjuncts can be adapted to provide the described functionalities for other surgical staplers, and other surgical instruments.

The present invention can be practiced by employing conventional materials, methodology and equipment. Accordingly, the details of such materials, equipment and methodology are not set forth herein in detail. In the previous descriptions, numerous specific details are set forth, such as specific materials, structures, chemicals, processes, etc., in order to provide a thorough understanding of the present invention. However, it should be recognized that the present invention can be practiced without resorting to the details specifically set forth. In other instances, well known processing structures have not been described in detail, in order not to unnecessarily obscure the present invention.

Only an exemplary embodiment of the present invention and but a few examples of its versatility are shown and described in the present disclosure. It is to be understood that the present invention is capable of use in various other combinations and environments and is capable of changes or modifications within the scope of the inventive concept as expressed herein.

## Claims

1. A sensing adjunct to a surgical stapler (101; 201; 301; 901; 1001; 1101), the sensing adjunct comprising:
at least one sensor (219, 231; 409; 509; 609, 619; 715, 717; 809, 811; 911; 1005; 1121) comprising an optical sensor and configured to generate a signal indicative of a property of a subject tissue and/or to sense mechanical or biological properties and to operate independently of the surgical stapler (101; 201; 301; 901; 1001; 1101);
wherein the sensing adjunct is coupled to the surgical stapler (101; 201; 301; 901; 1001; 1101) or is configured as a standalone substitutive component acting to serve as a replacement for a component of the surgical stapler (101; 201; 301; 901; 1001; 1101);
**characterized in that**
the at least one sensor (219, 231; 409; 509; 609, 619; 715, 717; 809, 811; 911; 1005; 1121) is configured to be modulated to obtain measurements corresponding to multiple tissue depths from a sensor surface by varying at least one of intensity, wavelength, and light emitter to receiver spacing.

2. The sensing adjunct of claim 1, wherein the at least one sensor further (219, 231; 409; 509; 609, 619; 715, 717; 809, 811; 911; 1005; 1121) comprises one of a chemical sensor, a mechanical sensor, an electrical sensor and an acoustic sensor.

3. The sensing adjunct of claim 2, wherein the sensing adjunct takes the form of a surgical stapler anvil (113; 213; 313; 401; 501; 601; 701; 801; 1113) that serves to act in place of a non-sensing surgical stapler anvil or wherein the sensing adjunct takes the form of a shell that optionally couples to the anvil or body of a surgical stapler (101; 201; 301; 901; 1001; 1101).

4. The sensing adjunct of claim 2 or 3, wherein said at least one further sensor (219, 231; 409; 509; 609, 619; 715, 717; 809, 811; 911; 1005; 1121) is configured to measure at least one of tissue interaction force, tissue compression force, and tissue tension.

5. The sensing adjunct of claim 4, comprising force or pressure sensing elements mechanically coupled to a rigid substrate; wherein tissue interaction force at the working surface is mechanically transmitted to the sensor elements by pins or a pressurized cavity; or comprising a pressure sensitive layer consisting essentially of a piezoelectric, resistive, or capacitive film, sheet or coating.

6. The sensing adjunct of claim 1 or 2, wherein the sensing adjunct acts independently, or is communicatively coupled via a wireless connection to at least one of another sensing device and a base station.

7. The sensing adjunct of claim 2 or 3, wherein the at least one sensor (219, 231; 409; 509; 609, 619; 715, 717; 809, 811; 911; 1005; 1121) comprises optical emitters and receivers which are coupled optically through light guides or optical fibers to a working surface of the surgical stapler (101; 201; 301; 901; 1001; 1101).

8. The sensing adjunct of any one of claims 1 to 3, wherein the sensing adjunct comprises a sensor configured to quantify staple gap or relative change thereof.

9. The sensing adjunct of claim 8, comprising an actuated staple gap adjustment module that is configured to vary the staple gap responsive to at least one of user, or sensor input, optionally wherein the sensor input includes tissue oxygenation, compression pressure, or staple gap height.

10. The sensing adjunct of claim 1 with the capability to deliver or assist in delivering therapy.

11. The sensing adjunct of any one of claims 1 or 2 or 3 or 9, further comprising an actuator configured for assisting with firing staples of the surgical stapler (101; 201; 301; 901; 1001; 1101), optionally wherein the actuator which fires is responsive to at least one of user input, or sensor based input.

12. The sensing adjunct of claim 1 or 2, wherein said at least one sensor (219, 231; 409; 509; 609, 619; 715, 717; 809, 811; 911; 1005; 1121) comprises at least one optical sensor configured to measure at least one of tissue oxygenation, oxygen delivery, oxygen utilization, tissue characterization, and tissue general health using oximetry, or spectroscopic techniques, and at least one of tissue perfusion, tissue flow dynamics, tissue oxygen content, tissue chemical composition, tissue immunologic activity, tissue pathogen concentration, tissue water content, tissue neoplastic cell content, or tissue dysplastic cell content using fluorescence based techniques.

13. The sensing adjunct of claim 1 or 12, wherein said at least one sensor (219, 231; 409; 509; 609, 619; 715, 717; 809, 811; 911; 1005; 1121) is configured to assess oxygen quantity in said tissue utilizing time-resolved techniques for evaluating oxygen dependent quenching of optical radiation.

14. The sensing adjunct of any one of the preceding claims, wherein said at least one sensor (219, 231; 409; 509; 609, 619; 715, 717; 809, 811; 911; 1005; 1121) provides readings configured to provide information for maintaining consistency of procedure.

15. The sensing adjunct of claim 1, wherein the sensing adjunct is communicatively coupled with at least one reference sensor (135; 1301), which provides a baseline measurement of at least one of a physiologic property of tissue and a mechanical property of tissue.

16. A surgical instrument comprising an adjunct of any one of the preceding claims.

17. The surgical instrument of claim 16, wherein the surgical instrument comprises a surgical stapler (101; 201; 301; 901; 1001; 1101) or a compression anastomosis ring.

18. The surgical instrument of claim 17, comprising
a surgical stapler (101; 201; 301; 901; 1001; 1101) augmented with sensing capabilities comprising an anvil (113; 213; 313; 401; 501; 601; 701; 801; 1113) and/or housing provided with or in the form of said sensing adjunct, the sensing adjunct being coupled to a communication interface (125; 1325) in turn coupled to a controller, and
one or more reference sensors (135; 1301) communicating to communication interface (125; 1325) and configured to be placed on one or more portions of the tissue to serve as a baseline measurement or determine tissue viability, or suitability for a particular procedure.

## Patentansprüche

1. Detektionszubehör für ein chirurgisches Klammergerät (101; 201; 301; 901; 1001; 1101), wobei das Detektionszubehör umfasst:
wenigstens einen Sensor (219, 231; 409; 509; 609, 619; 715, 717; 809, 811; 911; 1005; 1121), welcher einen optischen Sensor umfasst und dazu eingerichtet ist, ein Signal zu erzeugen, welches für eine Eigenschaft eines Subjekt-Gewebes indikativ ist, und/oder mechanische oder biologische Eigenschaften zu detektieren und unabhängig von dem chirurgischen Klammergerät (101; 201; 301; 901; 1001; 1101) zu arbeiten;
wobei das Detektionszubehör mit dem chirurgischen Klammergerät (101; 201; 301; 901; 1001; 1101) gekoppelt ist oder als eine eigenständige, substitutive Komponente eingerichtet ist, welche derart wirkt, dass sie als ein Ersatz für eine Komponente des chirurgischen Klammergeräts (101; 201; 301; 901; 1001; 1101) dient;
**dadurch gekennzeichnet, dass**
der wenigstens eine Sensor (219, 231; 409; 509; 609, 619; 715, 717; 809, 811; 911; 1005; 1121) dazu eingerichtet ist, moduliert zu werden, um durch ein Variieren wenigstens eines aus Intensität, Wellenlänge und LichtSender-zu-Empfänger-Abstand Messungen zu erhalten, welche von einer Sensoroberfläche aus verschiedenen Gewebetiefen entsprechen.

2. Detektionszubehör nach Anspruch 1, wobei der wenigstens eine Sensor (219, 231; 409; 509; 609, 619; 715, 717; 809, 811; 911; 1005; 1121) ferner eines aus einem chemischen Sensor, einem mechanischen Sensor, einem elektrischen Sensor und einem akustischen Sensor umfasst.

3. Detektionszubehör nach Anspruch 2, wobei das Detektionszubehör in der Form eines Chirurgisches-Klammergerät-Ambosses (113; 213; 313; 401; 501; 601; 701; 801; 1113) vorliegt, welcher dazu dient, anstelle eines Nicht-Detektions-Chirurgisches-Klammergerät-Ambosses zu wirken, oder wobei das Detektionszubehör in der Form einer Hülle vorliegt, welche optional mit dem Amboss oder dem Körper eines chirurgischen Klammergeräts (101; 201; 301; 901; 1001; 1101) gekoppelt ist.

4. Detektionszubehör nach Anspruch 2 oder 3, wobei der wenigstens eine Sensor (219, 231; 409; 509; 609, 619; 715, 717; 809, 811; 911; 1005; 1121) ferner dazu eingerichtet ist, wenigstens eines aus einer Gewebeinteraktionskraft, einer Gewebekompressionskraft und einer Gewebespannung zu messen.

5. Detektionszubehör nach Anspruch 4, umfassend Kraft- oder Druckdetektionselemente, welche mechanisch mit einem starren Substrat gekoppelt sind; wobei eine Gewebeinteraktionskraft an der Arbeitsoberfläche durch Stifte oder einen unter Druck stehenden Hohlraum mechanisch an die Sensorelemente übertragen wird; oder umfassend eine druckempfindliche Schicht, welche im Wesentlichen aus einer piezoelektrischen, resistiven oder kapazitiven Folie, Lage oder Beschichtung besteht.

6. Detektionszubehör nach Anspruch 1 oder 2, wobei das Detektionszubehör unabhängig wirkt oder über eine drahtlose Verbindung kommunikativ mit wenigstens einem aus einer anderen Detektionsvorrichtung und einer Basisstation gekoppelt ist.

7. Detektionszubehör nach Anspruch 2 oder 3, wobei der wenigstens eine Sensor (219, 231; 409; 509; 609, 619; 715, 717; 809, 811; 911; 1005; 1121) optische Sender und Empfänger umfasst, welche durch Lichtführungen oder optische Fasern optisch mit einer Arbeitsoberfläche des chirurgischen Klammergeräts (101; 201; 301; 901; 1001; 1101) gekoppelt sind.

8. Detektionszubehör nach einem der Ansprüche 1 bis 3, wobei das Detektionszubehör einen Sensor umfasst, welcher dazu eingerichtet ist, einen Klammerspalt oder eine relative Änderung davon zu quantifizieren.

9. Detektionszubehör nach Anspruch 8, umfassend ein betätigtes Klammerspalt-Einstellmodul, welches dazu eingerichtet ist, den Klammerspalt als Reaktion auf wenigstens eines aus einer Benutzereingabe oder einem Sensoreingang zu variieren, optional wobei der Sensoreingang eine Gewebesauerstoffanreicherung, einen Kompressionsdruck oder eine Klammerspalthöhe umfasst.

10. Detektionszubehör nach Anspruch 1 mit der Fähigkeit, eine Therapie abzugeben oder dabei zu helfen, eine Therapie abzugeben.

11. Detektionszubehör nach einem der Ansprüche 1 oder 2 oder 3 oder 9, ferner umfassend einen Aktuator, welcher dazu eingerichtet ist, bei einem Abfeuern von Klammern des chirurgischen Klammergeräts (101; 201; 301; 901; 1001; 1101) zu helfen, optional wobei der abfeuernde Aktuator auf wenigstens eines aus einer Benutzereingabe oder einem sensorbasierten Eingang reagiert.

12. Detektionszubehör nach Anspruch 1 oder 2, wobei der wenigstens eine Sensor (219, 231; 409; 509; 609, 619; 715, 717; 809, 811; 911; 1005; 1121) wenigstens einen optischen Sensor umfasst, welcher dazu eingerichtet ist, unter Verwendung von Oxymetrie oder spektroskopischen Techniken wenigstens eines aus einer Gewebesauerstoffanreicherung, einer Sauerstoffzufuhr, einer Sauerstoffverwertung, einer Gewebecharakterisierung und einer allgemeinen Gesundheit des Gewebes sowie unter Verwendung fluoreszenzbasierter Techniken wenigstens eines aus einer Gewebedurchblutung, einer Gewebeflussdynamik, einem Gewebesauerstoffgehalt, einer chemischen Zusammensetzung des Gewebes, einer immunologischen Aktivität des Gewebes, einer Gewebeerregerkonzentration, eines Gewebewassergehalts, eines Gehalts an neoplastischen Zellen in dem Gewebe oder eines Gehalts an dysplastischen Zellen in dem Gewebe zu messen.

13. Detektionszubehör nach Anspruch 1 oder 12, wobei der wenigstens eine Sensor (219, 231; 409; 509; 609, 619; 715, 717; 809, 811; 911; 1005; 1121) dazu eingerichtet ist, unter Verwendung zeitaufgelöster Techniken zur Einschätzung eines sauerstoffabhängigen Abfangens optischer Strahlung eine Sauerstoffmenge in dem Gewebe abzuschätzen.

14. Detektionszubehör nach einem der vorhergehenden Ansprüche, wobei der wenigstens eine Sensor (219, 231; 409; 509; 609, 619; 715, 717; 809, 811; 911; 1005; 1121) Messwerte bereitstellt, welche dazu eingerichtet sind, Information zur Beibehaltung der Konsistenz des Verfahrens bereitzustellen.

15. Detektionszubehör nach Anspruch 1, wobei das Detektionszubehör kommunikativ mit wenigstens einem Referenzsensor (135; 1301) gekoppelt ist, welcher eine Basismessung wenigstens eines aus einer physiologischen Gewebeeigenschaft und einer mechanischen Gewebeeigenschaft bereitstellt.

16. Chirurgisches Instrument, umfassend ein Zubehör nach einem der vorhergehenden Ansprüche.

17. Chirurgisches Instrument nach Anspruch 16, wobei das chirurgische Instrument ein chirurgisches Klammergerät (101; 201; 301; 901; 1001; 1101) oder einen Kompressionsanastomosering umfasst.

18. Chirurgisches Instrument nach Anspruch 17, umfassend
ein chirurgisches Klammergerät (101; 201; 301; 901; 1001; 1101), welches mit Detektionsfähigkeiten erweitert ist, umfassend einen Amboss (113; 213; 313; 401; 501; 601; 701; 801; 1113) und/oder ein Gehäuse, welches mit dem Detektionszubehör bereitgestellt ist oder in der Form davon vorliegt, wobei das Detektionszubehör mit einer Kommunikationsschnittstelle (125; 1325) gekoppelt ist, welche wiederum mit einer Steuerung gekoppelt ist, und
einen oder mehrere Referenzsensoren (135; 1301), welche mit der Kommunikationsschnittstelle (125; 1325) kommunizieren und dazu eingerichtet sind, an einem oder mehreren Abschnitten des Gewebes platziert zu sein, um als eine Basismessung zu dienen oder eine Gewebeviabilität oder -eignung für ein bestimmtes Verfahren zu bestimmen.

## Revendications

1. Dispositif de détection pour une agrafeuse chirurgicale (101 ; 201 ; 301 ; 901 ; 1001 ; 1101), le dispositif de détection comprenant :
au moins un capteur (219, 231 ; 409 ; 509 ; 609, 619 ; 715, 717 ; 809, 811 ; 911 ; 1005 ; 1121) comprenant un capteur optique et configuré pour générer un signal indicatif d'une propriété d'un tissu sujet et/ou pour capter des propriétés mécaniques ou biologiques et pour fonctionner indépendamment de l'agrafeuse chirurgicale (101 ; 201 ; 301 ; 901 ; 1001 ; 1101);
le dispositif de détection étant accouplé à l'agrafeuse chirurgicale (101 ; 201 ; 301 ; 901 ; 1001 ; 1101) ou étant configuré comme composant substitutif autonome agissant pour servir de remplacement à un composant de l'agrafeuse chirurgicale (101 ; 201 ; 301 ; 901 ; 1001 ; 1101) ;
**caractérisé en ce que**
ledit au moins un capteur (219, 231 ; 409 ; 509 ; 609, 619 ; 715, 717 ; 809, 811 ; 911 ; 1005 ; 1121) est configuré pour être modulé afin d'obtenir les mesures correspondant aux multiples profondeurs de tissu depuis une surface du capteur en faisant varier au moins l'un parmi l'intensité, la longueur d'onde, et la distance entre l'émetteur et le récepteur de lumière.

2. Dispositif de détection selon la revendication 1, dans lequel le au moins un capteur (219, 231 ; 409 ; 509 ; 609, 619 ; 715, 717 ; 809, 811 ; 911 ; 1005 ; 1121) comprend en outre l'un parmi un capteur chimique, un capteur mécanique, un capteur électrique et un capteur acoustique.

3. Dispositif de détection selon la revendication 2, dans lequel le dispositif de détection prend la forme d'une enclume d'agrafeuse chirurgicale (113 ; 213 ; 313 ; 401 ; 501 ; 601 ; 701 ; 801 ; 1113) qui sert pour agir à la place d'une enclume d'agrafeuse chirurgicale non de détection ou le dispositif de détection prenant la forme d'une enveloppe qui s'accouple éventuellement à l'enclume ou au corps d'une agrafeuse chirurgicale (101 ; 201 ; 301 ; 901 ; 1001 ; 1101).

4. Dispositif de détection selon la revendication 2 ou 3, dans lequel ledit au moins un capteur (219, 231 ; 409 ; 509 ; 609, 619 ; 715, 717 ; 809, 811 ; 911 ; 1005 ; 1121) est configuré pour mesurer au moins l'une d'une force d'interaction tissulaire, d'une force de compression tissulaire, et d'une tension de tissu.

5. Dispositif de détection selon la revendication 4, comprenant des éléments de détection de force ou de pression mécaniquement accouplés à un substrat rigide ; la force d'interaction tissulaire au niveau de la surface de travail étant mécaniquement transmise aux éléments de détection par des épingles ou une cavité sous pression ; ou comprenant une couche sensible à la pression essentiellement constituée d'un film, d'une feuille ou d'un revêtement piézoélectrique, résistif, ou capacitif.

6. Dispositif de détection selon la revendication 1 ou 2, le dispositif de détection agissant indépendamment, ou étant accouplé de manière à communiquer par l'intermédiaire d'une connexion sans fil à au moins un de un autre dispositif de détection et d'une station de base.

7. Dispositif de détection selon la revendication 2 ou 3, le au moins un capteur (219, 231 ; 409 ; 509 ; 609, 619 ; 715, 717 ; 809, 811 ; 911 ; 1005 ; 1121) comprenant des émetteurs et des récepteurs optiques qui sont optiquement accouplés à travers des guides de lumière ou des fibres optiques à une surface de travail de l'agrafeuse chirurgicale (101 ; 201 ; 301 ; 901 ; 1001 ; 1101).

8. Dispositif de détection selon l'une quelconque des revendications 1 à 3, le dispositif d'adjonction de détection comprenant un capteur configuré pour quantifier l'espace d'agrafeuse ou son changement relatif.

9. Dispositif de détection selon la revendication 8, comprenant un module d'ajustement d'espace d'agrafeuse déclenché qui est configuré pour faire varier l'espace d'agrafeuse sensible à au moins l'un parmi l'entrée d'un utilisateur ou d'un capteur, éventuellement l'entrée d'un capteur comprenant l'oxygénation tissulaire, la pression de compression, ou la hauteur de l'espace d'agrafeuse.

10. Dispositif de détection selon la revendication 1 ayant la capacité d'administrer ou d'accompagner l'administration d'une thérapie.

11. Dispositif de détection selon l'une quelconque des revendications 1 ou 2 ou 3 ou 9, comprenant en outre un dispositif d'actionnement configuré pour accompagner le déclenchement des agrafes de l'agrafeuse chirurgicale (101 ; 201 ; 301 ; 901 ; 1001 ; 1101), éventuellement le dispositif d'actionnement qui déclenche étant sensible à au moins l'un parmi l'entrée utilisateur, ou l'entrée basée sur le capteur.

12. Dispositif de détection selon la revendication 1 ou 2, dans lequel ledit au moins un capteur (219, 231 ; 409 ; 509 ; 609, 619 ; 715, 717 ; 809, 811 ; 911 ; 1005 ; 1121) comprend au moins un capteur optique configuré pour mesurer au moins l'un parmi l'oxygénation tissulaire, l'administration d'oxygène, l'utilisation d'oxygène, la caractérisation de tissu, et la santé générale d'un tissu en utilisant l'oxymétrie, ou les techniques spectroscopiques, et au moins l'une parmi la perfusion tissulaire, la dynamique d'écoulement tissulaire, la teneur en oxygène du tissu, la composition chimique du tissu, l'activité immunologique du tissu, la concentration du tissu en pathogènes, la teneur en eau du tissu, la teneur du tissu en cellules néoplasiques, ou la teneur du tissu en cellules dysplasiques en utilisant les techniques basées sur la fluorescence.

13. Dispositif de détection selon la revendication 1 ou 12, ledit au moins un capteur (219, 231 ; 409 ; 509 ; 609, 619 ; 715, 717 ; 809, 811 ; 911 ; 1005 ; 1121) étant configuré pour évaluer la quantité d'oxygène dans ledit tissu en utilisant des techniques à résolution temporelle pour évaluer l'extinction du rayonnement optique en fonction de l'oxygène.

14. Dispositif de détection selon l'une quelconque des revendications précédentes, ledit au moins un capteur (219, 231 ; 409 ; 509 ; 609, 619 ; 715, 717 ; 809, 811 ; 911 ; 1005 ; 1121) fournissant des lectures configurées pour fournir des informations destinées au maintien de la cohérence de la procédure.

15. Dispositif de détection selon la revendication 1, le dispositif d'adjonction de détection étant accouplé de manière à pouvoir communiquer avec au moins un capteur de référence (135 ; 1301), qui fournit une mesure de ligne de référence d'au moins l'une parmi une propriété physiologique du tissu et une propriété mécanique du tissu.

16. Instrument chirurgical comprenant un dispositif selon l'une quelconque des revendications précédentes.

17. Instrument chirurgical selon la revendication 16, l'instrument chirurgical comprenant une agrafeuse chirurgicale (101 ; 201 ; 301 ; 901 ; 1001 ; 1101) ou un anneau d'anastomose par compression.

18. Instrument chirurgical selon la revendication 17, comprenant
une agrafeuse chirurgicale (101 ; 201 ; 301 ; 901 ; 1001 ; 1101) augmentée de capacités de détection comprenant une enclume (113 ; 213 ; 313 ; 401 ; 501 ; 601 ; 701 ; 801 ; 1113) et/ou un logement fourni avec ou sous la forme dudit dispositif de détection, le dispositif de détection étant accouplé à une interface de communication (125 ; 1325) à son tour accouplée à un dispositif de commande, et un ou plusieurs capteurs de référence (135 ; 1301) communiquant avec une interface de communication (125 ; 1325) et configurés pour être placés sur une ou plusieurs parties du tissu afin de servir de mesure de ligne de référence ou de déterminer la viabilité du tissu, ou l'adaptabilité à une procédure particulière.
